## Europäisches Patentamt

## European Patent Office

## Office européen des brevets

(19)

(11) Publication number: **0 110 559**
**B1**

(12)

# EUROPEAN PATENT SPECIFICATION

(45) Date of publication of patent specification: **15.07.87**

(51) Int. Cl.⁴: **C 07 C 121/52, C 07 C 120/04**

(21) Application number: **83306539.4**

(22) Date of filing: **27.10.83**

(54) **Process for the preparation of 4-chloro-2-nitrobenzonitrile.**

(30) Priority: **26.11.82 JP 206020/82**

(43) Date of publication of application:
**13.06.84 Bulletin 84/24**

(45) Publication of the grant of the patent:
**15.07.87 Bulletin 87/29**

(84) Designated Contracting States:
**CH DE FR GB IT LI**

(56) References cited:
**CH-A- 514 554**
**DE-A-2 126 720**
**GB-A- 949 619**

(73) Proprietor: **SANWA KAGAKU KENKYUSHO CO., LTD.**
**No. 35, Higashi-sotobori-cho**
**Higashi-ku Nagoya-shi Aichi-ken (JP)**

(72) Inventor: **Kurono, Masayasu**
**2-72-2, Kakeage-cho Minami-ku**
**Nagoya-shi Aichi-ken (JP)**
Inventor: **Yamaguchi, Takuji**
**174-1, Higashikata Minami Monzen-cho**
**Kuwana-shi Mie-ken (JP)**
Inventor: **Usui, Toshinao**
**45, Minami Uzura 5-chome**
**Gifu-shi Gifu-ken (JP)**

(74) Representative: **Diamond, Bryan Clive et al**
**Gee & Co. Chancery House Chancery Lane**
**London WC2A 1QU (GB)**

Courier Press, Leamington Spa, England.

## Description

The present invention relates to a novel process for the preparation of 4-chloro-2-nitrobenzonitrile.

The compound is a useful intermediate for preparing various pharmaceutical agents and other chemicals, for instance it is important as an intermediate for synthesizing 5-(4-chloro-5-sulphamoyl-2-thenylaminophenyl)-1H-tetrazole which is useful as a diuretic agent.

Hitherto, it has been known that the compound can be prepared by heating 4-chloro-2-nitrobenzoic acid with methane sulfonamide and phosphorus pentachloride (see DE—OS—21 26 720). This conventional method gives a good yield but, since the starting material 4-chloro-2-nitrobenzoic acid is expensive, this method is costly when used industrially.

An object of the invention is, therefore, to provide a novel process for the preparation of 4-chloro-2-nitrobenzonitrile, which is suitable for industrial production, since it uses a starting material available commercially at a reasonable price.

Another object of the invention is to provide a novel process for the preparation of 4-chloro-2-nitrobenzonitrile, which gives a satisfactory yield.

The starting material (2,5-dichloronitrobenzene) has been known as a raw material for making dyes and is available commercially in large amounts and at a reasonable price.

To obtain a nitrile compound from a corresponding aromatic halide, a method of using copper (I) cyanide in the presence or absence of a solvent has generally been adopted, and in this case some reports indicated the advantage for success for such reaction of adding a small amount of an inorganic cyanide. It is to be expected, however, that if there is an electron-attractive group such as a nitro group in the *ortho* position of the starting compound, an undesirable side reaction proceeds and thus a high yield of the desired compound cannot be expected.

Thus GB—A—949 619 discloses reacting 2,3-dichloronitrobenzene with a mixture of copper (I) cyanide and an alkali metal cyanide such as KCN in amount of up to 70 mol% of the CuCN, at a temperature of 140° to 240°C and in the presence of a nitrogenous base such as pyridine or an alkyl-substituted pyridine in amount of up to 0.5 mol per mol of the dichloronitrobenzene, to prepare 2-chloro-6-nitrobenzonitrile. There is no disclosure of preparation of the *4-chloro* compound.

It is also known from CH—A—514 554, to react a chloro-substituted 2-nitrobenzonitrile with copper (I) cyanide at 100° to 220°C for 1 to 10 hours in an inert organic solvent such as dimethylacetamide. The only Example is of producing 4-methyl-2-nitrobenzonitrile.

Both these processes replace a 2-chloro atom by a 2-cyano group; but we have found that 4-chloro-2-nitrobenzonitrile could only be produced by these processes in low yield since numerous by-products are formed.

According to the invention we provide a process for the preparation of 4-chloro-2-nitrobenzonitrile, which comprises reacting 2,5-dichloronitrobenzene with copper (I) cyanide and in the presence of an inorganic cyanide, characterized in that the reaction is carried out in N,N-dimethyl-formamide as inert solvent and at a temperature of 140° to 170°C for 3.0 to 6.0 hours.

Preferred conditions for obtaining a good yield are 1.0 to 1.5 molar amount of copper (I) cyanide to 1.0 molar amount of 2,5-dichloronitro-benzene, and most preferably in equimolar amount, in the presence of 0.01 molar amount of potassium cyanide and 0.9 molar amount of N,N-dimethylformamide at a temperature of 160° to 170°C.

Suitable inorganic cyanides are potassium cyanide and sodium cyanide.

The desired 4-chloro-2-nitrobenzonitrile can be obtained in a higher yield by pouring the resulting reaction mixture into cooled toluene, stirring the solution at room temperature, filtering off insoluble inorganic compounds, distilling off the solvent in the filtrate and then washing the residue with carbon tetrachloride. The 4-chloro-2-nitrobenzonitrile obtained by said simple post-treatment contains almost no impurity.

The invention will now be further illustrated by reference to Examples.

Example 1 4-Chloro-2-nitrobenzonitrile

A mixture of 230.4 g (1.20 mol) of 2,5-dichloronitrobenzene, 108 g (1.20 mol) of copper (I) cyanide, and 0.80 g (0.012 mol) of potassium cyanide in 80 ml (1.04 mol) of N,N-dimethylformamide was heated for 5.5 hours at 165 to 170°C.

The reaction mixture was poured slowly into 1.2 l of cold toluene and followed by stirring for 13 hours at room temperature. The precipitate was filtered and washed with 100 ml of ethyl acetate. After concentration of the combined filtrate, the residue was washed with 140 ml of carbon tetrachloride to give 125 g of 4-chloro-2-nitrobenzonitrile as pale yellow crystals. From the solution of carbon tetrachloride, 35 g more 4-chloro-2-nitrobenzonitrile were obtained. Total yield was 160 g (73.1%).

m.p. 90~93°C

| | |
|---|---|
| IR spectrum: $\gamma_{max}^{KBr}$ cm$^{-1}$ | 2240 (—CN), 1525 (—NO$_2$) 1350 (—NO$_2$) |
| NMR spectrum ppm (CDCl$_3$) | 8.00 (2H, s, aromatic 5-H, 6-H) 8.47 (1H, s, aromatic 3-H) |
| MS spectrum m/e | 182 (M$^+$) |

2

Examples 2 to 6

The procedure of Example 1 was repeated, with variation of the conditions as shown in Table 1.

TABLE 1

| Example | 2,5-Dichloro-nitrobenzene (mole) | Copper (I) cyanide (mole) | Inorganic cyanide (mole) | | N,N-Dimethyl-formamide (mole) | Reaction time (hr) | Reaction temperature (°C) | Yield of 4-Chloro-2-nitro-benzonitrile (%) |
|---------|---------|---------|---------|---------|---------|---------|---------|---------|
| 2 | 1 | 1.0 | 1.0 | (NaCN) | 5.0 | 12 | reflux | 56.0 |
| 3 | 1 | 1.0 | 0.1 | (NaCN) | 5.0 | 12 | " | 47.0 |
| 4 | 1 | 1.0 | 0.1 | (KCN) | 5.0 | 6 | " | 70.0 |
| 5 | 1 | 1.0 | 0.01 | (KCN) | 3.0 | 5 | " | 70.2 |
| 6 | 1 | 1.1 | 0.055 | (KCN) | 3.0 | 5 | " | 43.0 |

**0 110 559**

### Claims

1. A process for the preparation of 4-chloro-2-nitrobenzonitrile, which comprises reacting 2,5-dichloronitrobenzene with copper (I) cyanide and in the presence of an inorganic cyanide, characterized in that the reaction is carried out in N,N-dimethylformamide as inert solvent and at a temperature of 140° to 170°C for 3.0 to 6.0 hours.

2. A process as claimed in Claim 1, wherein said copper (I) cyanide is added in 1.0 to 1.5 molar amount based on the 2,5-dichloronitrobenzene.

3. A process as claimed in Claim 1 or 2, wherein said inorganic cyanide is added in 0.01 to 0.1 molar amount based on the 2,5-dichloronitrobenzene.

4. A process as claimed in any preceding Claim, which is followed by the step of pouring the resulting reaction mixture into cold toluene, filtering-off insoluble materials, distilling the filtrate to dryness and washing the residue with carbon tetrachloride.

### Patentansprüche

1. Verfahren zum Herstellung von 4-Chlor-2-Nitrobenzonitril durch Umsetzung von 2,5-Dichlornitrobenzol mit Kupfer-(I)-Cynanid in Gegenwart eines anorganischen Cyanids, dadurch gekennzeichnet, dass die Reaktion mit N,N-Dimethylformamid als inertes Lösungsmittel und bei einer Temperatur von 140° bis 170°C während 3 bis 6 Stunden durchgeführt wird.

2. Verfahren nach Anspruch 1, dadurch gekennzeichnet, dass das Kupfer-(I)-Cyanid in einer Molarmenge von 1,0 bis 1,5 basierend auf dem 2,5-Dichlornitrobenzol zugefügt wird.

3. Verfahren nach Anspruch 1 oder 2, dadurch gekennzeichnet, dass das anorganische Cyanid in einer Molarmenge von 0,01 bis 0,1 basierend auf dem 2,5-Dichlornitrobenzol zugefügt wird.

4. Verfahren nach einem der vorangehenden Ansprüche, dadurch gekennzeichnet, dass dem Verfahren eine weitere Stufe folgt, in welcher die Reaktionsmischung in kaltes Toluen gegeben wird, die unlöslichen Stoffe ausfiltriert werden, das Filtrat zur Trockene destilliert und der Rückstand mit Kohlenstoff-Tetrachlorid gewaschen wird.

### Revendications

1. Procédé de préparation de 4-chloro-2-nitrobenzonitrile par réaction de 2,5-dichloro-nitrobenzène avec du cyanure de cuivre (I) en présence d'un cyanure inorganique, caractérisé en ce que la réaction est effectuée dans du N,N-diméthyl-formamide comme solvant inerte et à une température de 140° à 170°C pendant 3 à 6 heures.

2. Procédé suivant la revendication 1, caractérisé en ce que le cyanure de cuivre (I) est ajouté en quantité molaire de 1,0 à 1,5 basée sur le 2,5-dichloronitrobenzène.

3. Procédé suivant la revendication 1 ou 2, caractérisé en ce que le cyanure inorganique est ajouté en quantité molaire de 0,01 à 0,1 basée sur le 2,5-dichloro-nitrobenzène.

4. Procédé suivant l'une quelconque des revendications précédentes, caractérisé en ce qu'il est suivi d'une étape où le mélange réactif est versé dans du toluène froid, les substances insolubles sont éliminées par filtration, le filtrat est distillé à siccité et le résidu lavé avec du tetrachlorure du carbone.